(19) **Europäisches Patentamt / European Patent Office / Office européen des brevets**

(11) **EP 1 520 036 B1**

(12) **EUROPEAN PATENT SPECIFICATION**

(45) Date of publication and mention
of the grant of the patent:
**10.10.2007 Bulletin 2007/41**

(21) Application number: **03761599.4**

(22) Date of filing: **30.06.2003**

(51) Int Cl.:
*C12Q 1/48* (2006.01)          *C12Q 1/70* (2006.01)

(86) International application number:
**PCT/EP2003/050279**

(87) International publication number:
**WO 2004/003227 (08.01.2004 Gazette 2004/02)**

(54) **NEW MUTATIONAL PROFILES IN HIV-1 REVERSE TRANSCRIPTASE CORRELATED WITH PHENOTYPIC DRUG RESISTANCE**

NEUE MUTATIONSMUSTER IN HIV-1 REVERSER TRANSCRIPTASE, DIE MIT PHENOTYPISCHER RESISTENZ GEGEN MEDIKAMENTE KORRELIEREN

NOUVEAUX PROFILS MUTATIONNELS DANS LA TRANSCRIPTASE INVERSE DU VIH-1 CORRELES A LA RESISTANCE PHENOTYPIQUE AUX MEDICAMENTS

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.07.2002 US 393025 P**

(43) Date of publication of application:
**06.04.2005 Bulletin 2005/14**

(73) Proprietor: **Tibotec Pharmaceuticals Ltd.**
**Eastgate**
**Little Island**
**Co Cork (IE)**

(72) Inventors:
• **AZIJN, Hilde**
  **B-3001 Leuven (BE)**
• **DE BETHUNE, Marie-Pierre T.M.M.G**
  **B-3078 Everberg (BE)**
• **VINGERHOETS, Johan, Hendrika, Jozef**
  **B-2110 Wijnegem (BE)**

(74) Representative: **Daelemans, Frank F.R.**
**Tibotec-Virco Comm. VA**
**J&J Patent Law Department**
**Generaal De Wittelaan L 11B 3**
**2800 Mechelen (BE)**

(56) References cited:
WO-A-00/73511          WO-A-00/78996
WO-A-02/33402          WO-A-97/27332

• STEIN CHRISTIAN A ET AL: "Sequence analysis of proviral HIV RT amplified directly by a semi-quantitative technique from AZT treated patients" JOURNAL OF MEDICAL VIROLOGY, vol. 44, no. 2, 1994, pages 115-121, XP009018682 ISSN: 0146-6615
• SCHINAZI R F ET AL: "Mutations in retroviral genes associated with drug resistance: 2000-2001 update" INTERNATIONAL ANTIVIRAL NEWS 2000 UNITED KINGDOM, vol. 8, no. 5, 2000, pages 65-91, XP009018670 ISSN: 0965-2310

**Description**

The present invention is directed to the field of nucleic acid diagnostics and the identification of base variation in target nucleic acid sequences. The invention provides novel mutations or mutational profiles of HIV-1 reverse transcriptase gene correlated with a phenotype causing alterations in sensitivity to anti-HIV drugs. The present invention also relates to the use of genotypic characterization of a target population of HIV and the subsequent association, i.e. correlation, of this information to phenotypic interpretation in order to correlate virus mutational profiles with drug resistance. The invention further relates to methods of utilizing the mutational profiles of the invention in databases, drug development, *i.e.,* drug design, and drug modification, therapy and treatment design and clinical management.

The development and standardization of plasma HIV-1 RNA quantification assays has led to the use of viral load measurements as a key therapy response monitoring tool. The goal of antiretroviral therapy is to reduce plasma viremia to below the limit of detection on a long-term basis. However, in a significant number of patients, maximal suppression of virus replication is not achieved and for those in whom this goal is reached, a significant number experience viral load rebound. Viral load data provide no information on the cause of the failure.

Therapy failure may be due to a number of factors, including insufficient antiviral activity of the regimen, individual variations in drug metabolism and pharmacodynamics, difficulties in adhering to dosing regimen, requirements for treatment interruption due to toxicity, and viral drug resistance. Moreover, drug resistance may develop in a patient treated with sub-optimal antiretroviral therapy or a patient may be infected with drug-resistant HIV-1. Although drug resistance may not be the primary reason for therapy failure, in many cases any situation which permits viral replication in the presence of an inhibitor sets the stage for selection of resistant variants.
Viral drug resistance can be defined as any change in the virus that improves replication in the presence of an inhibitor. HIV-1 drug resistance was first described in 1989 and involved patients that had been treated with zidovudine monotherapy (Larder, B.A., et al., Science 243, 1731-1734 (1989)). Emergence of resistance is almost always being observed during the course of treatment of patients with single antiretroviral drugs. Similarly, *in vitro* passage of viral cultures through several rounds of replication in the presence of antiretroviral compounds leads to the selection of viruses whose replication cycle is no longer susceptible to the antiretroviral compounds used. Resistance development has also been observed with the introduction of dual nucleoside reverse transcriptase inhibitors (NRTI) combination therapy as well as during the administering of the more potent non- nucleoside reverse transcriptase inhibitors (NNRTIs), protease inhibitors (PIs) and combinations thereof. Individual antiretroviral agents differ in the rate at which resistance develops: selection for resistant variants may occur within weeks of treatment or resistance may emerge after a longer treatment period.

Extensive genetic analysis of resistant viral isolates generated through *in vivo* or *in vitro* selection has revealed that resistance is generally caused by mutations at some specific site(s) of the viral genome. The mutational patterns that have been observed and reported for HIV-1 and that are correlated with drug resistance are very diverse: some antiretroviral agents require only one single genetic change, while others require multiple mutations for resistance to appear. A summary of mutations in the HIV genome correlated with drug resistance has been compiled (See e.g. Schinazi, Int. Antiviral News. 6, 65 (2000)). Electronic listings with mutations are available at different web locations such as hiv-web.lanl.gov/content/index, www.hivb.stanford.edu, and www.hivresistanceweb.com. Document WO 00/73511 A disclose mutation 194Q in the reverse transcriptase of patients treated with AZT.

A genetic mutation is normally written in reference to the wild type virus, *i.e.*, K101N refers to replacement of a Lysine at codon 101 with a Asparagine (The Molecular biology of the Cell, 1994,Garland Publishing, NY). However, the mutations of the invention do not depend on the wild-type example listed in order to be within the practice of the invention. For example, the mutation 101N, refers to an Asparagine at the 101 codon regardless of the whether there was a Lysine at 101 prior to mutation. Alternatively, it may be said that a particular amino acid occurs at a given position, wherein "position" is equivalent to "codon". Mutations can also be identified in nucleic acids such as RNA, DNA, mRNA. The degree of susceptibility of a genetic variant to an antiretroviral compound is expressed herein relative to the wild-type virus (HIV IIIB/LAI reference sequence) as found, for example, in GenBank, (K03455, gi 327742, M38432). An alteration in viral drug sensitivity is defined as a change in resistance or a change in susceptibility of a viral strain to said drug. Susceptibilities are generally expressed as ratios of $EC_{50}$ or $EC_{90}$ values (the $EC_{50}$ or $EC_{90}$ value being the drug concentration at which 50% or 90% respectively of the viral population is inhibited from replicating) of a viral strain under investigation compared to the wild type strain. Hence, the susceptibility of a viral strain can be expressed as a fold change in susceptibility, wherein the fold change is derived from the ratio of for instance the $EC_{50}$ values of a mutant viral strain compared to the wild type. In particular, the susceptibility of a viral strain or population may also be expressed as resistance of a viral strain, wherein the result is indicated as a fold increase in $EC_{50}$ as compared to wild type $EC_{50}$.

As antiretroviral drugs are administered for longer periods, mostly in combination with each other, and as new antiretrovirals are being developed and added to the present drugs, new resistance-correlated genetic variants are being identified. Of particular importance is that the combination of antiretroviral agents can influence resistance characteristics.

Once viral resistance has developed, salvage therapy options may be severely restricted due to cross-resistance within each drug class. This is as important for initial treatment as for when a therapy change is called for in order to minimize the emergence of resistance and improve the long-term prognosis of the patient. The choice of therapy regimen will be supported by knowledge of the resistance profile of the circulating virus population. Additionally, therapy combinations will have a greater chance of being effective if they include agents that have a demonstrated potential of suppressing a particular virus population.

A number of applications describe the occurrence of mutations in HIV and their correlation to the development of drug resistance (WO 00/73511; WO 02/33402; WO 02/22076; WO 00/78996). The instant invention adds to the art mutations in the reverse transcriptase gene and their correlation i.e. association to viral drug resistance

## DETAILED DESCRIPTION OF THE INVENTION

The knowledge that the mutation 194G correlates with a fold change in resistance can be applied in certain useful methods. The present invention relates to methods for evaluating the effectiveness of a reverse transcriptase inhibitor, based on the presence of at least one mutation 194G in HIV reverse transcriptase. The presence of said mutation correlates to a fold change in susceptibility or resistance of an HIV-1 viral strain towards at least one reverse transcriptase drug. The effectiveness of a reverse transcriptase inhibitor in the presence of at least one of said mutations may be determined using e.g. enzymatic, phenotypic and genotypic methods. The correlation between the mutational profiles in HIV-1 reverse transcriptase and drug usage may be useful for clinical toxicological and forensic applications. A combined approach involving genotypic and phenotypic resistance testing to correlate mutations with resistance phenotypes may be used. More in particular, the present invention provides a correlation between at least one strain of HIV-1 having at least one 194G mutation in HIV-1 reverse transcriptase and a fold change in resistance.

The effectiveness of a reverse transcriptase inhibitor as an antiviral therapy for a patient infected with at least one HIV-1 strain comprising mutant reverse trascriptase may be determined using a method comprising: (i) determining whether a sample collected from an HIV-1-infected patient comprises a HIV reverse transcriptase having at least one mutation 194G; and (ii) correlating the presence of said at least one mutation of step (i) to a change in effectiveness of said reverse transcriptase inhibitor

In general a change in effectiveness can be expressed as a fold change in resistance. The fold change may be determined using a cellular assay including the cytopathogenic assay or the Antivirogram® (WO 97/27480). Alternatively, the fold change in susceptibility may be derived from database analysis such as the VirtualPhenotype™ (WO 01/79540). A decrease in susceptibility vis-à-vis the wild type virus correlates to an increased viral drug resistance, and hence reduced effectiveness of said drug. To determine the viral drug susceptibility the activity of the mutant enzyme may be compared to the activty of a wild type enzyme. In phenotyping assays pseudotyped viruses may be used. The mutations present in HIV-1 reverse transcriptase may be determined at the nucleic acid or amino acid level using sequencing or hybridization techniques. A report may be generated that shows the region of the patient virus that has been sequenced, including at least one mutation 194G. The report may include antiretroviral drugs, drug(s) for which a known resistance-associated mutation has been identified and/or to what extent the observed mutation(s) selected from at least 194G are indicative of resistance to drugs. The sample to be evaluated can be a bodily fluid including blood, serum, plasma, saliva, urine, or a tissue including gut tissues.

The fact that particular data correlate, indicates that a causal relationship exits between the data. Hence, a particular result renders a particular conclusion more likely than other conclusions.

A drug effective against mutant HIV-1 reverse transcriptase may be identified by a method, comprising: (i) providing a nucleic acid comprising HIV-1 reverse transcriptase comprising at least one mutation 194G; (ii) determining a phenotypic response to said drug for said HIV-1 recombinant virus; and (iii) identifying a drug effective against mutant HIV based on the phenotypic response of step (ii) .The nucleic acid comprising HIV-1 of step (i) may be recombined into a proviral nucleic acid deleted for said sequence to generate a recombinant HIV-1 virus.

Identifying a drug is defined as making a selection of drugs clinically available based on the effectiveness of said drug. In addition to the selection of clinically available drugs identifying also relates to the selection of clinical drug candidates. The phenotypic response may be determined using cellular assays such as the Antivirogram®. An effective drug against mutant HIV-1 comprising at least one mutation 194G in reverse transcriptase is defined as a drug having a phenotypic response expressed, as e.g. a fold change in susceptibility lower than a defined cut-off that may be determined for a drug.

An other useful method for identifying, a drug effective against mutant HIV-1 reverse transcriptase comprises: (i) providing a HIV-1 reverse transcriptase comprising at least one mutation 194G, (ii) determining the activity of said drug on said HIV-1 reverse transcriptase for said drug; (iii) determining the activity of said drug on wild type HIV-1 reverse transcriptase; (iii) determining the ratio of the activity determined in step (iii) over the activity determined in step (ii); (v) identifying an effective drug against mutant HIV based on the ratio of step (iv). A ratio lower than a defined cut-off value that can be specific for said drug is indicative that the drug is effective against mutant HIV-1 (WO 02/33402).

The activity of said drug on mutant HIV-1 reverse transcriptase having at least one mutation 194G, can be determined in an enzymatic assay, wherein the mutant reverse transcriptase, is compared to the wild type enzyme by its enzymatic characteristics (e.g. Maximal velocity ($V_{max}$), Michaelis-Menten constant ($K_m$), catalytic constant ($k_{cat}$)) (Antimicrob. Agents Chemotherap. 1989 33(12), 2109-2114; Antimicrob. Agents Chemotherap. 1989 33(10), 1729-1734; Anal Biochem. 1996, 235(2) 141-152). An activity means any output generated by the assay including fluorescence, fluorescence polarization, luminiscence, absorbance, radioactivity, resonance energy transfer mechanisms, magnetism.

The response of a mutant HIV reverse transcriptase having at least one mutation 194G may be expressed as viral fitness (WO 00/78994). This viral fitness can be defined as the ability of a viral strain to replicate in the presence or absence of a component, such as a reverse transcriptase inhibitor. This viral fitness is dependent on a combination of factors including viral factors which include mutations occurring in viral proteins, host factors which include immune responses, differential expression of membrane proteins and selective pressures which include the presence of antiviral agents such as reverse transcriptase inhibitors.

Interestingly, the reverse transcriptase inhibitors that can be used in the present methods include Zidovudine, Nevirapine, Efavirenz, Abacavir, Capravirine, Lamivudine, Didanosine, Stavudine, Adefovir, Zalcitabine, Delavirdine, DPC-086, DPC-083, Tenofovir, and compound 1 (Benzonitrile, 4-[[6-amino-5-bromo-2-[(4-cyanophenyl)-amino]-4-pyrimidinyl]oxy]-3,5-dimethyl-, compound 1). In particular, the reverse transcriptase inhibitor is selected from Nevirapine, Efavirenz, Capravirine, DPC-086 and compound 1. Suitably, the inhibitor is selected from compound 1, Nevirapine and Efavirenz.

Conveniently, the methods of the present invention are performed using samples of an HIV-infected patient that has been treated with at least a reverse transcriptase inhibitor. More in particular, the patient contains mutant viruses bearing at least one additional mutation at position in the HIV reverse transcriptase selected from 41, 62, 65, 67, 69, 70, 74, 75, 98, 100, 101, 103, 106, 108, 116, 118, 138, 151, 178, 181, 184, 188, 190 , 210, 215, 219, 225, 227, 230, 234, 236, 238, and 318. Even more in particular, the mutant viruses are resistant towards the therapy the patient is taken.

A vector comprising an HIV-1 sequence having at least one mutation 194G in the HIV-1 reverse transcriptase gene may be useful for the phenotypic analysis. The present knowledge about the correlation between a fold change in susceptibility and the presence of at least one mutation 194G in HIV-1 reverse transcriptase can be used to prepare an isolated and purified HIV-1 reverse transcriptase sequence having at least one mutation 194G.

The knowledge of the mutations of the present invention offers the possibility to develop probes and primers directed to said mutations. An isolated and purified oligonucleotide comprising a HIV-1 reverse transcriptase sequence of 5 to 100 bases comprising at least one mutation 194G, may be useful for in vitro diagnosis of viral drug resistance. Suitable oligonucleotides for nucleic acid amplifying technologies contain 5 to 35 nucleic acid bases. More suitably such oligonucleotide contains between 15 to 30 nucleic acid bases. An oligonucleotide may contain the mutant base at the 3' end so as to enable the detection of the mutant using PCR. Oligonucleotides may also be used as probes including molecular beacons (Tyagi, Nature Biotechnol 1998, 16(1) 49-53), and TaqMan probes.

A computer system directed by software wherein the software correlates the presence of at least one mutation in a human immunodeficiency virus type-1 (HIV-1) reverse transcriptase and fold change in susceptibility of at least one strain of HIV-1 to a reverse transcriptase inhibitor, characterized in that the software uses at least one record corresponding to a correlation between at least one mutation 194G, and treatment with at least a reverse transcriptase inhibitor can be used for evaluating resistance towards therapy.

A neural network that predicts the development of therapeutic agent resistance or sensitivity against at least one viral strain comprising at least one mutation 194G can be used (WO 01/95230).

Genotyping methodologies

Resistance of HIV to antiretroviral drugs may be determined at the genotypic level by identifying mutations in the HIV-1 genome and by inferring the resistance of HIV-1 to antiretroviral drugs through searching for mutational patterns known to correlate with resistance. Assays for detection of mutations in HIV-1 may be based on polymerase chain reaction (PCR) amplification of viral genomic sequences. These amplified sequences are then analyzed using either hybridization or sequencing techniques. Hybridization-based assays include primer-specific PCR, which makes use of synthetic oligonucleotides designed to allow selective priming of DNA synthesis. See Larder, B.A., et al., AIDS 5, 137-144 (1991); Richman, D.D., et al., J. Infect. Dis. 164, 1075-1081 (1991); Gingeras, T.R., et al., J. Infect. Dis. 164, 1066-1074 (1991). Only when primer sequences match the target sequence (wild-type or mutant) at the 3' end, is amplification of target sequences possible and DNA fragments are produced. Knowledge of the primer sequences allows one to infer the sequence of the viral isolate under investigation, but only for the region covered by the primer sequences. Other hybridization-based assays include differential hybridization (Eastman, P.S., et al., J. Acq. Imm. Def. Syndr. Human Retrovirol. 9, 264-273 (1995); Holodniy, M., et al., J. Virol. 69, 3510-3516 (1995); Eastman, P.S., et al., J. Clin. Micro. 33, 2777-2780 (1995).); Line Probe Assay (LiPAJ HIV-11 RT, Innogenetics) (Stuyver, L., et al., Antimicrob. Agents Chemotherap. 41, 284-291 (1997)); and biochip technology such as GENECHIP® technology (Affymetrix) (D'Aquila, R.T. Clin. Diagnost. Virol. 3, 299-316 (1995); Fodor, S.P.A. et al., Nature 364, 555-556 (1993); Fodor, S.P.A. Nature 227, 393-395 (1997). The sequence may also be determined using mass spectroscopic technologies. DNA sequencing assays provide information on all nucleotides of the sequenced region. Sequencing results may be reported as amino acid changes at positions in the protease gene and the reverse transcriptase gene compared to the wild-type reference sequence. The changes included in the genotyping report may be limited to mutations at positions known to manifest drug resistance-associated polymorphisms. Polymorphisms at positions not associated with drug resistance may be omitted.

Phenotyping methodologies

Phenotyping assays measure the ability of a replicating virus to grow in the presence of compounds compared to a wild-type reference virus such as e.g. HIV-1/LAI, HIV-1/NL4.3, HIV-1/HXB2 or e.g.HIV-2/ROD. Alternatively, phenotyping assays are performed with pseudotyped viruses not able to replicate. Consequently, these assays directly measure the degree of viral resistance or susceptibility to specific inhibitors. In this case, one measures the effect of all mutational interactions, the effects of genetic changes as yet unknown or not previously identified, the effect of the background genotype, etc., on the phenotype. Some phenotypic assays are discussed below.

*Cytopathic effect assay (CPE assay)*

Determination of the antiviral activity of a compound was done as described in Pauwels R. et al. (J Virol Methods 1988; 20(4):309-21). Various concentrations of the test compounds were brought into each well of a flat-bottom microtiter plate. Subsequently, HIV and MT4 cells were added to a final concentration of 200-250 50% cell culture infectious doses ($CCID_{50}$)/well and 30,000 cells/well, respectively. After 5 days of incubation (37°C, 5% $CO_2$), the cytopathic effect of the replicating virus was determined by the tetrazolium colorimetric MTT method. The dose protecting 50% of the cells from virus cytopathic effect was defined as the $EC_{50}$, while the dose achieving 90% protection was defined as the $EC_{90}$.

**Reporter gene assay**

The reporter gene assay used MT4-LTR-EGFP cells. Upon infection by HIV-1, the expression of the viral tat product increases transcription from the HIV-1 LTR promoter, leading to high-level expression of the reporter gene product.

The assay procedure was similar to the CPE assay, except for the end reading of the assay, which was performed on day 3 by measuring the relative fluorescence of treated cultures and comparing this with the relative fluorescence of untreated cultures. The $EC_{50}$ or the $EC_{90}$ of a compound was defined as the concentration that inhibited the relative fluorescence by 50% or 90% respectively.

*Antiviral assay with PBMC cultures*

The purification and activation of PBMCs as well as the antiviral assays were carried out as described (CDER. Guidance for Industry Points to Consider in the Preclinical Development of Antiviral Drugs. 1990). The assay measured the extent that a drug inhibits HIV p24 antigen production by peripheral blood mononuclear cells (PBMC) cultures acutely infected with a viral strain. The susceptibility determination uses phytohaemaglutinine (PHA)-stimulated PBMCs from normal donors. In the *in vitro* infection experiments 1000 $CCID_{50}$ per million PHA-stimulated PBMCs was used. Cultures were split 1/2 every 3 to 4 days and compound was added together with the addition of new medium.

The p24 antigen production was measured using a commercial kit, according to the manufacturer protocol (NEN), at the moment that the p24 production of untreated infected cultures is maximal; i.e. between 7 and 11 days after infection.

The % p24 production was calculated by means of following equation:

$$\%p24 = 100 \times \frac{[p24]_{Sample} - [p24]_{Mock\_Control}}{[p24]_{HIV\_Control} - [p24]_{Mock\_Control}}$$

where $[p24]_{Sample}$ is the p24 concentration in an infected treated culture, $[p24]_{HIV\_Control}$ is the p24 concentration in an infected untreated culture and $[p24]_{Mock\_Control}$ is the p24 concentration in a mock-infected culture. The dose achieving 50% p24 production according to the above formula was defined as the $EC_{50}$, while the dose achieving 10% p24 production according to the above formula was defined as the $EC_{90}$.

*Antiviral assay with monocytes/macrophages*

The assay measured the extent that a drug inhibits HIV p24 antigen production by primary monocytes/macrophages acutely infected with HIV-1/BaL (300 $CCID_{50}$/ml). The susceptibility determination used monocytes/macrophages isolated from PBMCs from normal donors by plastic adherence. Every 5 days cultures were fed with complete medium containing the appropriate compound concentrations. The p24 antigen production was measured at day 14 after virus challenge and $EC_{50}$ and $EC_{90}$ values were calculated.

*Recombinant virus assays*

A recombinant virus assay (RVA) starts with the amplification of viral target sequences by means of PCR. The amplicons are incorporated into a proviral laboratory clone deleted for the sequences, present in the amplicon. This generates a stock of chimeric viruses. The viruses are tested for their ability to grow in the presence of different concentrations of drugs. Results are obtained by calculating $EC_{50}$ values for each inhibitor and by reporting the results as $EC_{50}$ values, expressed in $\mu$M concentrations, or by computing the ratio of the $EC_{50}$ values found for the chimeric virus to the $EC_{50}$ values found for a wild type susceptible laboratory virus tested in parallel.

In the latter case, resistance is expressed as "fold-resistance" (fold change in susceptibility, FC) compared to a wild-type susceptible HIV-1 strain.

The use of reporter gene systems for susceptibility testing allows the implementation of laboratory automation and standardization (Pauwels, et al., J.Virol. Methods 20, 309-321 (1988); Paulous, S., et al., International Workshop on HIV Drug Resistance, Treatment Strategies and Eradication, St. Petersburg, Florida, USA. Abstr. 46 (1997); and Deeks, S. G., et al., 2nd International Workshop on HIV Drug Resistance and Treatment Strategies, Lake Maggiore, Italy. Abstr. 53 (1998)).

The Antivirogram® assay (Virco) (WO 97/27480) is based on homologous recombination of patient derived HIV-1 *gag*/PR/RT sequences into a proviral HIV-1 clone correspondingly deleted for the *gag*/PR/RT sequences. A similar assay (Phenosense® ViroLogic, WO 97/27319) is based on enzymatic ligation of patient-derived PR/RT sequences into a correspondingly deleted proviral vector carrying an indicator gene, luciferase, inserted in the deleted HIV-1 envelope gene. An other assay has been developed by Bioalliance (Phenoscript, WO 02/38792). The development of high-throughput phenotyping and genotyping assays has allowed the establishment of a database containing the phenotypic resistance data and the genotypic sequences of over 30,000 clinical isolates.

**Experimental part**

Example 1. The Identification of Mutational Patterns in HIV-1 Reverse transcriptase and the Correlated Phenotypic Resistance.

Plasma samples from HIV-1-infected individuals from routine clinical practice were obtained and shipped to the laboratory on dry ice and stored at -70°C until analysis. Viral RNA was extracted from 200 $\mu$L patient plasma using the QIAAMP® Viral RNA Extraction Kit (Qiagen, Hilden, Germany), according to the manufacturers instructions. cDNA encompassing part of the *pol* gene was produced using Expand™ reverse transcriptase (Boehringer Mannheim). A 2.2kb fragment encoding the protease and RT regions were amplified from patient-derived viral RNA by nested polymerase chain reaction (PCR) using PCR primers and conditions as described. (Hertogs K., et al., Antimicrob. Agents Chemother. 42: 269-276 (1998), WO 01/81624). This genetic material was used in phenotyping and genotyping experiments.

Phenotypic analysis was performed using the recombinant virus assay (Antivirogram®)(WO 97/27480). MT-4 cells (Harada S., et al, Science 229: 563-566 (1985).) were co-transfected with pol gene PCR fragments and the protease-RT deleted HIV-1 molecular clone, pGEM3ΔPRT. This resulted in viable recombinant viruses containing protease/RT from the donor PCR fragment. After homologous recombination of amplicons into a PR-RT deleted proviral clone, the resulting recombinant viruses were harvested, titrated and used for *in vitro* susceptibility testing to antiretroviral drugs. The results of this analysis were expressed as fold change in susceptibility, reflecting the fold change in mean $EC_{50}$ ($\mu$M) of a particular drug when tested with patient-derived recombinant virus isolates, relative to the mean $EC_{50}$ ($\mu$M) of the same drug obtained when tested with a reference wild-type virus isolate (IIIB/LAI). Genotyping was performed by an automated population-based full-sequence analysis, through a dideoxynucleotide-based approach, using the BigDye™ terminator kit (Applied Biosystems, Inc.) and resolved on an ABI 377 DNA sequencer.

The genotypes are reported as amino acid changes at positions along the reverse transcriptasegene compared to the wild-type (HXB2) reference sequence. Analysis by VirtualPhenotype™ interpretational software (WO 01/79540) allowed detection of mutational patterns in the database containing the genetic sequences of the clinical isolates and linkage with the corresponding resistance profiles of the same isolates.

Example 2. Susceptibility analysis of HIV-1 variants constructed by site-directed mutagenesis

Mutations in the protease or RT coding region were created by site-directed mutagenesis, using the Quik-Change® Site-Directed Mutagenesis Kit (STRATAGENE®), of a wild-type HXB2-D *EcoRl-Pstl* restriction enzyme fragment, encompassing the HIV-1 *pol* gene and cloned into pGEM3 (Promega). All mutant clones were verified by DNA sequence analysis. PCR fragments were prepared from the mutated clones and the altered reverse transcriptasecoding regions were transferred into HIV-1 HXB2-D by homologous recombination as described above. The susceptibility of these recombinant viruses to drugs was determined by the MT-4 cell CPE protection assay.

Example 3. *In vitro* selection of resistant strains

Cells are infected at a high MOI (such as 1-50 $CCID_{50}$/cell), corresponding to > $10^9$ viral RNA copies / ml. These experiments have been designed to mimic the quasi-species variability that is observed in HIV infected individuals where $10^9$ to $10^{10}$ new viruses are produced daily with a mutation rate of $10^{-4}$ to $10^{-5}$. The infected MT4-LTR-EGFP cells are treated with inhibitors at 40, 200 nM, 1 $\mu$M and higher for a maximum of 30 days. The cultures are sub-cultivated and scored on virus-induced fluorescence and cytopathicity every 3 - 4 days. If full virus breakthrough (100% CPE) is observed the supernatants was collected and stored (new virus strain). If no full CPE was observed the cells were sub-cultivated and further grown in the presence of the same concentration compound till full virus breakthrough, with a maximum of 30 days. From the emerging virus populations a virus stock was grown in the absence of compounds and titrated. The sensitivities of the isolated strains to HIV-1 RT inhibitors were determined and the strains were genotyped.

*In vitro* drug selection experiments starting from wild-type HIV-1/LAI under pressure of compound 1 have been performed. Table 1 shows the genotypic and phenotypic characterization of the selected strains.

**Table 1 Characterization of the strains isolated from HIV-1/LAI in the presence of compound 1**

| | | | | High MOI | High MOI | High MOI | High MOI | High MOI |
|---|---|---|---|---|---|---|---|---|
| Strain | | LAI | SM26 | SM26 | SM26 | SM26 | SM26 | SM26 |
| Compound | | | - | Compound 1 | Compound 1 | Compound 1 | Compound 1 | Compound 1 |
| Concentration | | | | 0.040 $\mu$M | 0.200 $\mu$M | 1.000 $\mu$M | 0.200 $\mu$M | 1.000 $\mu$M |
| Days to breakthrough | | | | day 7 | day 7 | day 11 | day 9 | day 13 |
| Mutations present at start | | | K103N Y181C | K103N Y181C | K103N Y181C | K103N Y181C | K103N Y181C | K103N Y181C |
| Additional mutations | | | | E194E/K | L100I V189V/I E194G/E | L100I E194G | L100I V189V/I E194G/E R356R/K | L100I V189I E194G |
| **Compound** | | | | | | | | |
| Compound 1 | EC50 | 0.0014 | 0.0050 | 0.0078 | 0.1486 | 0.6682 | 0.0268 | 0.2614 |
| | FC | 1 | 4 | 6 | 106 | 477 | 19 | 187 |
| Efavirenz | EC50 | 0.0010 | 0.0367 | 0.0327 | 2.7470 | 6.7270 | 0.0565 | 3.5953 |
| | FC | 1 | 37 | 33 | 2.747 | 6.727 | 57 | 3.595 |
| Nevirapine | EC50 | 0.0763 | > 10.0000 | > 10.0000 | > 10.0000 | > 10.0000 | > 10.0000 | > 10.0000 |
| | FC | 1 | >131 | >131 | >131 | >131 | >131 | >131 |

The *in vitro* antiviral activity of compound 1 and current reverse transcriptase inhibitors against the selected strains was evaluated in acutely infected MT4 cells. Median $EC_{50}$ values together with the fold change in resistance (expressed as a ration of $EC_{50}$) as compared to wild type (FC) are reported.

**Claims**

1. A computer system directed by software wherein the software correlates the presence of at least one mutation in a human immunodeficiency virus type 1 (HIV-1) reverse transcriptase and a change in susceptibility of at least one strain of HIV-1 to a reverse transcriptase inhibitor, **characterized in that** the software uses at least one record corresponding to a correlation between at least one mutation 194G in said reverse transcriptase, and treatment with at least a reverse transcriptase inhibitor.

2. An in vitro method for evaluating the effectiveness of a reverse transcriptase inhibitor as an antiviral therapy for a patient infected with at least one mutant HIV-1 strain comprising:

    (i) determining whether a sample collected from an HIV-infected patient comprises a nucleic acid encoding HIV-1 reverse transcriptase having at least one mutation 194G;
    (ii) correlating the presence of said at least one mutation of step (i) to a change in effectiveness of said reverse transcriptase inhibitor.

3. A method for identifying a drug effective against mutant HIV-1 reverse transcriptase, comprising:

    (i) providing a HIV-1 reverse transcriptase nucleic acid comprising at least one mutation 194G;
    (ii) recombining said nucleic acid of step (i) into a proviral nucleic acid deleted for said sequence to generate a recombinant HN-1 virus;
    (iii) determining a phenotypic response of said drug to said HIV-1 reverse transcriptase; and
    (iv) identifying a drug effective against mutant HIV-1 based on the phenotypic response of step (iii).

4. A method for identifying a drug effective against mutant HIV-1 reverse transcriptase, comprising:

    (i) providing a HIV-1 reverse transcriptase comprising at least one mutation 194G;
    (ii) determining the activity of said drug on said HIV-1 reverse transcriptase;
    (iii) determining the activity of said drug on wild type HN-1 reverse transcriptase;
    (iv) determining the ratio of the activity determined in step (iii) over the activity determined in step (ii);
    (v) identifying an effective drug against mutant HIV-1 based on the ratio of step (iv).

5. An in vitro method for evaluating a change in viral drug susceptibility comprising:

    (i) determining whether a sample collected from an HIV-infected patient comprises a HIV-1 reverse transcriptase having at least one mutation 194G;
    (ii) correlating the presence of said at least one mutation of step (i) to a change in viral drug susceptibility.

6. A method for evaluating a change in viral drug susceptibility, comprising:

    (i) providing an HIV-1 comprising a reverse transcriptase comprising at least one mutation 194G;
    (ii) determining a phenotypic response of said virus to said drug; and
    (iii) correlating the phenotypic response of step (ii) to a change in viral drug susceptibility.

7. A method for evaluating a change in drug effectiveness against mutant HIV-1 reverse transcriptase, comprising:

    (i) providing a HIV-1 reverse transcriptase comprising at least one mutation 194G;
    (ii) determining the activity of said drug on said reverse transciptase;
    (iii) determining the activity of said drug on wild type HIV-1 reverse transcriptase and;
    (iv) determining the ratio of the activity determined in step (iii) over the activity determined in step (ii);
    (v) identifying an effective drug against mutant HIV-1 based on the ratio of step (iv).

8. A vector for performing phenotypic analysis comprising an HN-1 sequence having at least one mutation coding for 194G in the HIV reverse transcriptase.

9. An isolated and purified HIV-1 reverse transcriptase sequence having at least one mutation 194G, wherein said at least one mutation in said sequence correlates to a fold change in susceptibility towards a HIV-1 reverse transcriptase inhibitor.

**10.** An isolated and purified oligonucleotide comprising a HIV-1 reverse transcriptase sequence of 5 to 100 bases for in vitro diagnosis of viral drug resistance, **characterized in that** said oligonucleotide comprises at least one mutation coding for 194G.

**Patentansprüche**

**1.** Computersystem, welches durch eine Software gesteuert wird, wobei die Software die Anwesenheit mindestens einer Mutation in einer Reversen Transkriptase eines menschlichen Immunschwächevirus Typ 1 (HIV-1) und eine Änderung der Empfindlichkeit mindestens eines Stammes von HIV-1 gegenüber einem Reverse-Transkriptase-Inhibitor korreliert, **dadurch gekennzeichnet, dass** die Software mindestens einen Datensatz verwendet, der einer Korrelation zwischen mindestens einer Mutation 194G in der Reversen Transkriptase und der Behandlung mit mindestens einem Reverse-Transkriptase-Inhibitor entspricht.

**2.** In-Vitro-Verfahren zur Bewertung der Wirksamkeit eines Reverse-Transkriptase-Inhibitors als antivirale Therapie für einen Patienten, der mit mindestens einem mutanten HIV-1-Stamm infiziert ist, umfassend:

(i) Bestimmen, ob eine von einem HIVinfizierten Patienten erhaltene Probe eine Nukleinsäure umfasst, die HIV-1-Reverse Transkriptase mit mindestens einer Mutation 194G kodiert;
(ii) Korrelieren der Anwesenheit der mindestens einen Mutation in Stufe (i) mit einer Veränderung der Wirksamkeit des Reverse-Transkriptase-Inhibitors.

**3.** Verfahren zum Identifizieren eines Arzneimittels, das gegen mutante HIV-1-Reverse Transkriptase wirksam ist, umfassend:

(i) Bereitstellen einer HIV-1-Reverse-Transkriptase-Nukleinsäure, umfassend mindestens eine Mutation 194G;
(ii) Rekombinieren der Nukleinsäure aus Stufe (i) zu einer proviralen Nukleinsäure, bei der die Sequenz deletiert worden ist, um ein rekombinantes HIV-1-Virus zu erzeugen;
(iii) Bestimmen einer phänotypischen Reaktion des Arzneimittels auf die HIV-1-Reverse Transkriptase und
(iv) Identifizieren eines Arzneimittels, das gegen mutantes HIV-1 wirksam ist, auf Basis der phänotypischen Reaktion von Stufe (iii).

**4.** Verfahren zum Identifizieren eines Arzneimittels, das gegen mutante HIV-1-Reverse Transkriptase wirksam ist, umfassend:

(i) Bereitstellen einer HIV-1-ReverseTranskriptase, umfassend mindestens eine Mutation 194G;
(ii) Bestimmen der Wirksamkeit des Arzneimittels auf die HIV-1-Reverse Transkriptase;
(iii) Bestimmen der Wirksamkeit des Arzneimittels auf HIV-1-Reverse Transkriptase vom Wildtyp;
(iv) Bestimmen des Verhältnisses der in Stufe (iii) bestimmten Wirksamkeit zu der in Stufe (ii) bestimmten Wirksamkeit;
(v) Identifizieren eines wirksamen Arzneimittels gegen mutantes HIV-1 auf Basis des Verhältnisses von Stufe (iv).

**5.** In-Vitro-Verfahren zur Bewertung einer Veränderung der Empfindlichkeit gegenüber viralem Arzneimittel, umfassend:

(i) Bestimmen, ob eine von einem HIVinfizierten Patienten erhaltene Probe eine HIV-1-Reverse Transkriptase mit mindestens einer Mutation 194G umfasst;
(ii) Korrelieren der Anwesenheit der mindestens einen Mutation in Stufe (i) mit einer Veränderung der Empfindlichkeit gegenüber viralem Arzneimittel.

**6.** Verfahren zur Bewertung einer Veränderung der Empfindlichkeit gegenüber viralem Arzneimittel, umfassend:

(i) Bereitstellen eines HIV-1, umfassend eine Reverse Transkriptase, umfassend mindestens eine Mutation 194G;
(ii) Bestimmen einer phänotypischen Reaktion des Virus auf das Arzneimittel und
(iii) Korrelieren der phänotypischen Reaktion von Stufe (ii) auf eine Veränderung der Empfindlichkeit gegenüber viralem Arzneimittel.

7. Verfahren zur Bewertung einer Veränderung der Arzneimittelwirksamkeit gegen mutante HIV-1-Reverse Transkriptase, umfassend:

(i) Bereitstellen einer HIV-1-Reversen Transkriptase, umfassend mindestens eine Mutation 194G;
(ii) Bestimmen der Wirksamkeit des Arzneimittels auf die Reverse Transkriptase;
(iii) Bestimmen der Wirksamkeit des Arzneimittels auf HIV-1-Reverse Transkriptase vom Wildtyp und
(iv) Bestimmen des Verhältnisses der in Stufe (iii) bestimmten Wirksamkeit zu der in Stufe (ii) bestimmten Wirksamkeit;
(v) Identifizieren eines wirksamen Arzneimittels gegen mutante HIV-1 auf Basis des Verhältnisses von Stufe (iv).

8. Vektor zur Durchführung von phänotypischer Analyse, umfassend eine HIV-1-Sequenz mit mindestens einer für 194G kodierenden Mutation in der HIV-Reverse Transkriptase.

9. Isolierte und gereinigte HIV-1-Reverse-Transkriptase-Sequenz mit mindestens einer Mutation 194G, wobei die mindestens eine Mutation in der Sequenz mit einem Fold Change der Empfindlichkeit für einen HIV-1-Reverse-Transkriptase-Inhibitor korreliert.

10. Isoliertes und gereinigtes Oligonukleotid, umfassend eine HIV-1-Reverse-Transkriptase-Sequenz von 5 bis 100 Basen zur in-Vitro-Diagnose der Resistenz gegen virales Arzneimittel, **dadurch gekennzeichnet, dass** das Oligonukleotid mindestens eine für 194G kodierende Mutation umfasst.

## Revendications

1. Système informatisé commandé par un logiciel, dans lequel le logiciel met en corrélation la présence d'au moins une mutation dans une transcriptase inverse de virus d'immunodéficience humaine de type 1 (VIH-1) et un changement de la sensibilité d'au moins une souche de VIH-1 avec un inhibiteur de la transcriptase inverse, **caractérisé en ce que** le logiciel utilise au moins un enregistrement correspondant à une corrélation entre une mutation 194G au moins dans ladite transcriptase inverse et un traitement avec au moins un inhibiteur de la transcriptase inverse.

2. Procédé *in vitro* destiné à évaluer l'efficacité d'un inhibiteur de la transcriptase inverse comme thérapie antivirale dédiée à un patient infecté par au moins une souche mutante de VIH-1, comprenant les étapes consistant à :

(i) déterminer si un échantillon, prélevé sur un patient infecté par un VIH, comprend un acide nucléique codant pour une transcriptase inverse de VIH-1 ayant au moins une mutation 194G ;
(ii) établit la corrélation entre la présence de ladite mutation au moins de l'étape (i) et un changement de l'efficacité dudit inhibiteur de la transcriptase inverse.

3. Procédé d'identification d'une substance médicamenteuse efficace contre une transcriptase inverse de VIH-1 mutant, comprenant les étapes consistant à :

(i) fournir un acide nucléique de transcriptase inverse de VIH-1 comprenant au moins une mutation 194G ;
(ii) recombiner ledit acide nucléique de l'étape (i) dans un acide nucléique proviral, dont on a supprimé ladite séquence, pour générer un virus VIH-1 recombinant ;
(iii) déterminer une réponse phénotypique de ladite substance médicamenteuse à ladite transcriptase inverse de VIH-1 ; et
(iv) identifier une substance médicamenteuse efficace contre un VIH-1 mutant sur la base de la réponse phénotypique de l'étape (iii).

4. Procédé d'identification d'une substance médicamenteuse efficace contre une transcriptase inverse de VIH-1 mutant, comprenant les étapes consistant à :

(i) fournir une transcriptase inverse de VIH-1 comprenant au moins une mutation 194G ;
(ii) déterminer l'activité de ladite substance médicamenteuse sur ladite transcriptase inverse de VIH-1 ;
(iii) déterminer l'activité de ladite substance médicamenteuse sur une transcriptase inverse de VIH-1 de type sauvage ;
(iv) déterminer le rapport de l'activité déterminée à l'étape (iii) sur l'activité déterminée à l'étape (ii) ;
(v) identifier une substance médicamenteuse efficace contre un VIH-1 mutant sur la base du rapport de l'étape (iv).

5. Procédé *in vitro* destiné à évaluer un changement de la sensibilité virale aux médicaments, comprenant les étapes consistant à :

(i) déterminer si un échantillon, prélevé sur un patient infecté par un VIH, comprend une transcriptase inverse de VIH-1 ayant au moins une mutation 194G ;
(ii) établit la corrélation entre la présence de ladite mutation au moins de l'étape (i) et un changement de la sensibilité virale aux médicaments.

6. Procédé d'évaluation d'un changement de la sensibilité virale aux médicaments, comprenant les étapes consistant à :

(i) fournir un VIH-1 comprenant une transcriptase inverse, qui comprend au moins une mutation 194G ;
(ii) déterminer une réponse phénotypique dudit virus à ladite substance médicamenteuse ; et
(iii)établit la corrélation entre la réponse phénotypique de l'étape (ii) et un changement de la sensibilité virale aux médicaments.

7. Procédé d'évaluation d'un changement de l'efficacité médicamenteuse contre une transcriptase inverse de VIH-1 mutant, comprenant les étapes consistant à :

(i) fournir une transcriptase inverse de VIH-1 comprenant au moins une mutation 194G ;
(ii) déterminer l'activité de ladite substance médicamenteuse sur ladite transcriptase inverse ;
(iii)déterminer l'activité de ladite substance médicamenteuse sur une transcriptase inverse de VIH-1 de type sauvage ; et
(iv) déterminer le rapport de l'activité déterminée à l'étape (iii) sur l'activité déterminée à l'étape (ii) ;
(v) identifier une substance médicamenteuse efficace contre un VIH-1 mutant sur la base du rapport de l' étape (iv).

8. Vecteur destiné à effectuer une analyse phénotypique comprenant une séquence de VIH-1 qui a au moins une mutation codant pour une 194G dans la transcriptase inverse de VIH.

9. Séquence de transcriptase inverse de VIH-1 isolée et purifiée ayant au moins une mutation 194G, dans laquelle ladite mutation au moins dans ladite séquence est en corrélation avec un changement de la sensibilité envers un inhibiteur de la transcriptase inverse de VIH-1.

10. Oligonucléotide isolé et purifié comprenant une séquence de transcriptase inverse de VIH-1 de 5 à 100 bases destinée à un diagnostic *in vitro* de la résistance virale aux médicaments, **caractérisée en ce que** ledit oligonucléotide comprend au moins une mutation codant pour une 194G.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0073511 A
- WO 0233402 A
- WO 0222076 A
- WO 0078996 A
- WO 9727480 A
- WO 0179540 A

- WO 0078994 A
- WO 0195230 A
- WO 9727319 A
- WO 0238792 A
- WO 0181624 A

**Non-patent literature cited in the description**

- **LARDER, B.A. et al.** *Science,* 1989, vol. 243, 1731-1734
- **SCHINAZI.** *Int. Antiviral News,* 2000, vol. 6, 65
- *Antimicrob. Agents Chemotherap.,* 1989, vol. 33 (12), 2109-2114
- *Antimicrob. Agents Chemotherap.,* 1989, vol. 33 (10), 1729-1734
- *Anal Biochem.,* 1996, vol. 235 (2), 141-152
- **TYAGI.** *Nature Biotechnol,* 1998, vol. 16 (1), 49-53
- **LARDER, B.A. et al.** *AIDS,* 1991, vol. 5, 137-144
- **RICHMAN, D.D. et al.** *J. Infect. Dis.,* 1991, vol. 164, 1075-1081
- **GINGERAS, T.R. et al.** *J. Infect. Dis.,* 1991, vol. 164, 1066-1074
- **EASTMAN, P.S. et al.** *J. Acq. Imm. Def. Syndr. Human Retrovirol.,* 1995, vol. 9, 264-273
- **HOLODNIY, M. et al.** *J. Virol.,* 1995, vol. 69, 3510-3516
- **EASTMAN, P.S. et al.** *J. Clin. Micro.,* 1995, vol. 33, 2777-2780

- **STUYVER, L. et al.** *Antimicrob. Agents Chemotherap.,* 1997, vol. 41, 284-291
- **D'AQUILA, R.T.** *Clin. Diagnost. Virol.,* 1995, vol. 3, 299-316
- **FODOR, S.P.A. et al.** *Nature,* 1993, vol. 364, 555-556
- **FODOR, S.P.A.** *Nature,* 1997, vol. 227, 393-395
- **PAUWELS R. et al.** *J Virol Methods,* 1988, vol. 20 (4), 309-21
- **PAUWELS et al.** *J.Virol. Methods,* 1988, vol. 20, 309-321
- **PAULOUS, S. et al.** *International Workshop on HIV Drug Resistance, Treatment Strategies and Eradication,* 1997, vol. 46
- **DEEKS, S. G. et al.** *2nd International Workshop on HIV Drug Resistance and Treatment Strategies,* 1998, vol. 53
- **HERTOGS K. et al.** *Antimicrob. Agents Chemother.,* 1998, vol. 42, 269-276
- **HARADA S. et al.** *Science,* 1985, vol. 229, 563-566